(19) Europäisches Patentamt
European Patent Office
Office européen des brevets

(11) Veröffentlichungsnummer: **0 087 751**
**B1**

(12) **EUROPÄISCHE PATENTSCHRIFT**

(45) Veröffentlichungstag der Patentschrift: **29.05.85**

(51) Int. Cl.⁴: **C 07 K 5/02**, A 61 K 37/02

(21) Anmeldenummer: **83101768.6**

(22) Anmeldetag: **23.02.83**

(54) **Wasserlösliche Peptide sehr schwer löslicher Aminosäuren.**

(30) Priorität: **25.02.82 DE 3206810**

(43) Veröffentlichungstag der Anmeldung: **07.09.83 Patentblatt 83/36**

(45) Bekanntmachung des Hinweises auf die Patenterteilung: **29.05.85 Patentblatt 85/22**

(84) Benannte Vertragsstaaten: **AT CH DE GB LI SE**

(56) Entgegenhaltungen:
**G.R. PETTIT: "Synthetic Peptides", Band 1, Seite 101, Van Nostrand Reinhold Company, New York, USA**

(73) Patentinhaber: **Pfrimmer + Co., Pharmazeutische Werke Erlangen GmbH, Hofmannstrasse 26, D-8520 Erlangen (DE)**

(72) Erfinder: **Fürst, Peter, Prof. Dr., Universität Hohenheim Garbenstrasse 30, D-7000 Stuttgart 70 (DE)**
Erfinder: **Pfaender, Peter, Prof. Dr., Aichelestrasse 8, D-7000 Stuttgart 70 Plieningen (DE)**
Erfinder: **Fekl, Werner, Dr., Rühlstrasse 1a, D-8520 Erlangen (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

**EP 0 087 751 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Aminosäuren sind Bestandteile des Eiweißes. Sie werden dem Organismus u. a. durch eiweißhaltige Nahrung zugeführt und daraus im Magen-Darm-Trakt durch die Wirkung der Verdauungsenzyme freigelegt und dann resorbiert. Beim Ausfall oder bei Insuffizienz dieser Enzyme oder bei stark geschwächten Patienten, ferner in Situationen, bei denen der Abbau von körpereigenem Protein den Aufbau überwiegt, weiterhin im Falle von spezifischen Krankheitsbildern, z. B. chronischem Nierenversagen oder Leberinsuffizienz, welche hochgradige und weit über das Normalmaß hinausgehende Supplementation mit essentiellen Aminosäuren in geeigneten Proportionen erfordert, müssen die freien Aminosäuren mittels entsprechender Präparate oral zugeführt werden, um zum Aufbau körpereigenen Eiweißes unmittelbar zur Verfügung zu stehen.

In vielen Fällen schwerer Erkrankung ist die Ernährung auf oralem Wege nicht oder nur schwer möglich. Insbesondere bei postoperativen und posttraumatischen Zuständen und in schweren Fällen von Karzinomen, Verbrennungen, Infektionen, akutem und chronischem Nierenversagen, Leberinsuffizienz sowie langanhaltender Bewußtlosigkeit oder schweren Stoffwechselstörungen müssen die Patienten dann parenteral mit Infusionslösungen ernährt werden, wobei zur Aufrechterhaltung der Ernährung dem Organismus neben Kohlenhydraten und Fett insbesondere auch Aminosäuren parenteral zugeführt werden müssen. Wegen der negativen Stickstoffbilanz, besonders bei postoperativer und posttraumatischer Stoffwechsellage, ist die Versorgung mit Aminosäuren von besonderer Bedeutung, wobei das Angebot nicht nur die essentiellen Aminosäuren umfassen soll, die der Organismus nicht selbst synthetisieren kann, sondern auch die anderen Aminosäuren, damit der Verlust an körpereigenen Proteinen möglichst schnell durch erneuten Aufbau dieser Proteine wieder ausgeglichen werden kann.

Zu den wichtigen Aminosäuren, die hierbei dem Organismus zur Verfügung gestellt werden, gehören u. a. auch die in Wasser nur sehr schwer löslichen Aminosäuren Tyrosin, deren Zufuhr essentiell ist bei Nierenversagen, und Cystin, das essentiell ist bei Neugeborenen und Säuglingen. Wegen ihrer geringen Wasserlöslichkeit ist die Bioverfügbarkeit dieser Aminosäuren nur gering. In Aminosäure-Infusionslösungen sind sie ebenfalls aus diesen Gründen nicht oder nur in sehr geringer Menge vorhanden und applizierbar.

Es wurde nun überraschenderweise festgestellt, daß durch eine Bindung dieser schwerlöslichen Aminosäuren an die beiden Aminogruppen der Aminosäure Lysin sich Tripeptide herstellen lassen, die trotz des Gehalts dieser beiden Aminosäuren im Verhältnis 2 : 1 Mol Lysin dennoch eine gute Wasserlöslichkeit aufweisen. Die Aminosäure Lysin besitzt außer der primären Aminogruppe in $\alpha$-Stellung noch am endständigen C-Atom, d. h. in $\varepsilon$-Stellung, eine zweite primäre Aminogruppe, die substituierbar ist. An diese beiden Aminogruppen sind bei den neuartigen Tripeptiden der allgemeinen Formel I gemäß Anspruch 1 jeweils Tyrosin- oder Cystin-Reste peptidartig gebunden. Während das Tyrosin nur eine Löslichkeit von 0,384 g/L $H_2O$ aufweist, beträgt die Löslichkeit des $N^2$-Tyrosinyl-$N^6$-tyrosinyl-lysins 622 g/L $H_2O$. Das Tripeptid ist somit um den Faktor $1{,}62 \cdot 10^3$ besser löslich in Wasser als das freie Tyrosin.

Die oral oder parenteral applizierten wasserlöslichen Tripeptide der schwer löslichen Aminosäuren mit dem Lysin besitzen die Eigenschaft, daß sie im Organismus durch körpereigene Aminopeptidasen, wie Peptidhydrolasen, gespalten werden, so daß mit dieser Applikation die schwer löslichen Aminosäuren dem Organismus leicht zum Aufbau körpereigener Proteine in ausreichender Menge zur Verfügung gestellt werden können, wobei ihm gleichzeitig auch noch die essentielle Aminosäure Lysin zugeführt wird. Beim erfindungsgemäß verwendeten $N^2$-Cystinyl-$N^6$-cystinyl-lysin werden neben dem Lysin sogar vier Mole Cystein bereitgestellt, da das freigelegte Cystin bekanntlich durch hydrierende Enzyme an der Disulfidbrücke in 2 Mole Cystein gespalten wird. Dies ist insbesondere wichtig für Neugeborene und Säuglinge, weil für diese Patientengruppe Cystin/Cystein essentiell ist. Daher kann diese schwefelhaltige Aminosäure, die einen wichtigen Baustein der Keratine in Haut, Haaren und Nägeln darstellt, und zur Vernetzung der linearen Eiweißmoleküle beiträgt, in ausreichender Menge als leicht resorbierbares Derivat zur Verfügung gestellt werden.

Im allgemeinen werden die erfindungsgemäßen Peptide dem Organismus zusammen mit anderen Aminosäuren und gegegenenfalls anderen niederen Peptiden mittels Infusionslösung appliziert. Das $N^2$-Cystinyl-$N^6$-cystinyl-lysin kann hierbei in einer Menge von 0,1—10 g, vorzugsweise von 1—10 g, je Liter, das $N^2$-Tyrosinyl-$N^6$-tyrosinyl-lysin in einer Menge von 0,1—20 g, vorzugsweise von 2—20 g, je Liter Infusionslösung enthalten sein.

Durch Auflösen der gereinigten Peptide und der gewünschten Aminosäuren in Aqua dest. werden diese Infusionslösungen erhalten, die gegebenenfalls noch Mineralsalze und andere Stoffe für die parenterale Ernährung aufweisen können und sich leicht isotonisch einstellen lassen. Die Lösungen können in üblicher Weise hitzesterilisiert werden (z. B. 0,5 h, 120°C, pH=5,5—7,5), ohne Gefahr einer Veränderung der neuartigen Tripeptide, da diese sehr hitzestabil sind. Im Falle der Verwendung zusammen mit Glucose oder anderen Kohlenhydraten in Infusionslösungen für parenterale Ernährung sind diese jedoch erst nach der Sterilisierung oder kurz vor der Verwendung beim Patienten zuzufügen, um Nebenreaktionen der freien Aminogruppe des Peptids mit der Glucose bei der Hitzesterilisierung auszuschließen.

Aber auch für die orale Applikation sind diese wasserlöslichen Tripeptide geeignet und können gegebenenfalls zusammen mit Aminosäuren und anderen Peptiden als Granulat oder in Dragees verabreicht werden.

Die neuen Tripeptide enthalten vorzugsweise die Aminosäuren jeweils in ihrer physiologisch aktiven L-Form. Sowohl das $N^2$-Cystinyl-$N^6$-cystinyl-lysin als auch das $N^2$-Tyrosinyl-$N^6$-tyrosinyl-lysin lassen sich in einfacher Weise nach der N-Carboxy-Anhydrid-Methode herstellen, wobei die freie Aminogruppe der vorgelegten Aminosäuren mit dem jeweiligen N-Carboxy-anhydrid der anzuhängenden Aminosäuren reagiert; R. Hirschmann et al., J. Org. Chem., Bd. 32, 3415—3425 (1967); J. Am. Chem. Soc., Bd. 93, 2746—2754 (1971). Diese Methode ist sehr vielseitig anwendbar und läßt sich in automatischen Vorrichtungen durchführen, siehe P. Pfaender et al., Hoppe—Seyler's Z. Physiol. Chem., Bd. 354, S. 267—285 (1973), und DE-OS 2 416 941 sowie US-PS 3 951 741. Zur Herstellung der erfindungsgemäßen Tripeptide werden die N-Carboxy-anhydride des Cystins oder Tyrosins mit dem Lysin umgesetzt unter Anknüpfung der Aminosäuren an die beiden Aminogruppen des Lysins.

Bei der bekannten Synthesemethode unter Verwendung der N-Carboxy-Anhydrid-Aminosäure-Verbindungen wird in Gegenwart eines Boratpuffers gearbeitet. Da die für therapeutische Zwecke notwendige Abtrennung des erhaltenen Tripeptids von dem Boratpuffer Schwierigkeiten bereitet, kann man diese Synthese auch in Aqua dest. durchführen, was überraschenderweise mit guten Ausbeuten gelingt. Um es von Nebenprodukten, d. h. eventuell entstandenen höheren Peptiden abzutrennen, werden dann die Tripeptide chromatographisch gereinigt, z. B. unter Verwendung von Sephadex®-Säulen.

### Beispiel 1

(Herstellung von $N^2$-Cystinyl-$N^6$-L-cystinyl-L-lysin)

a) Da Cystin-bis-N-Carboxy-Anhydrid sich nicht nach der üblichen Phosgenmethode herstellen läßt, wurde die Phosphor-Tribromid-Methode (Ben-Ishai u. Katchalski, J. Amer. Chem. Soc., Bd. 74, S. 3688—3689 (1952) angewendet. Es wurden 7,8 g (≙ $1{,}535 \times 10^{-2}$ Mol) getrocknetes Di-Z-L-Cystin in 200 ml abs. Diethylether suspendiert und bei 30° C im Wasserbad unter Rühren mit 1,15 mL $PBr_3$ versetzt. Die Suspension wurde hierbei zunächst dickflüssig, nach 30 min jedoch wieder dünner. Nach 18 h Rühren unter Rückfluß wurde der Niederschlag abgenutscht, der Filterkuchen noch einmal in wenig Ether suspendiert und erneut 30 min gerührt. Danach wurde wieder abgenutscht und das gebildete Cystin-bis-N-Carboxyanhydrid mit wenig Ether gewaschen.

b) 1,875 g Lysin · HCl (≙ 1,5 g oder 0,0103 Mol freies Lysin) wurden in 400 mL $H_2O$ bidest. unter Rühren gelöst, wobei unter Stickstoff gearbeitet wurde. Nach dem Kühlen der Lösung auf 0° C und Einstellung auf pH 10,2 mit ca. 50% NaOH wurden 3,30 g Cystin-bis-N-carboxy-anhydrid (≙ 0,0113 Mol) bei 10 000 UpM in kleinen Anteilen zugegeben, um die pH-Schwankungen möglichst gering zu halten. Nach 15 min wurde zur Decarboxylierung des entstandenen Peptidcarbaminats mit ca. 50% $H_2SO_4$ auf pH 3,5 eingestellt, worauf das Reaktionsgemisch mit 1 N NaOH neutralisiert wurde. Anschließend wurde das Reaktionsgemisch durch Lyophilisation von Wasser befreit. Der Rückstand betrug 8,6 g. Das trockene Rohprodukt wurde in 20 mL $H_2O$ bidest. gelöst und nach Abzentrifugieren von einem geringen Rückstand (Cystin) auf die Sephadex®-G-Säule (2,5 cm × 250 cm) gegeben. Als Elutionsmittel diente 0,5% Essigsäure. Als Eluat wurden 6 Fraktionen erhalten, die dünnschichtchromatographisch untersucht wurden. Die 1. Fraktion enthielt reines Tripeptid, während die 5. und 6. Fraktion nur Cystin und Nebenprodukte enthielten. Die 2. und 4. Fraktion wurden zusammen erneut in Aqua dest. aufgenommen und erneut auf die Sephadex®-Säule gegeben. Insgesamt konnten 1,04 g $N^2$,$N^6$-Di-L-cystinyl, L-lysin erhalten werden, das noch etwas mit dem Dipeptid (Cystinyl-Lysin) verunreinigt war. Im allgemeinen ist dessen Abtrennung nicht nötig, da auch das Dipeptid vom Organismus verwertet wird. Das erhaltene Produkt zeigte eine Löslichkeit von 125 g/L $H_2O$ (20° C), während die Werte für Cystin 0,09 g/L $H_2O$ (20° C) und für Lysin 666,0 g/L $H_2O$ (20° C) betragen.

### Beispiel 2

(Herstellung von $N^2$-Tyrosinyl-$N^6$-L-tyrosinyl-L-lysin)

Es wurde ein durch Umsetzung von Phosgen mit Tyrosin in Tetrahydro-furan als Lösungsmittel in üblicher Weise hergestelltes Tyrosin-N-Carboxy-Anhydrid verwendet. Die Umsetzung mit dem Lysin wurde in der automatischen Vorrichtung, wie sie in der DE-OS 2 416 941 oder US-PS 3 951 741 beschrieben ist, in Gegenwart eines NA-Boratpuffers, pH 10,2, durchgeführt, wobei das Lysin in der Pufferlösung gelöst mit dem feinst gepulverten Tyrosin-N-Carboxy-Anhydrid im Mol-Verhältnis 1 : 2 umgesetzt wurde. Nach 5 min wurde mit ca. 50% $H_2SO_4$ auf pH 3,5 angesäuert und dadurch das gebildete Tripeptid-Carbaminat decarboxyliert, worauf mit ca. 50% NaOH neutralisiert wurde. Zur Abtrennung von Natriumborat und Reinigung des Tripeptids wurde das lyophilisierte Reaktionsgemisch mit Methanol extrahiert. Der eingedampfte Methanolextrakt wurde dann in Aqua dest. gelöst und analog, wie in Beispiel 1 beschrieben, chromatographisch an einer Sephadex®-G-10-Säule gereinigt. Das erhaltene $N^2$-Tyrosinyl-$N^6$-L-tyrosinyl-L-lysin wurde durch Säurehydrolyse und übliche ASAR-Analyse der daraus erhaltenen Aminosäuren (2 Mol Tyrosin, 1 Mol Lysin) charakterisiert.

Das erhaltene $N^2$-Tyrosinyl-$N^6$-L-tyrosinyl-L-lysin läßt sich als Tyrosinquelle den Aminosäure-Infusionslösungen wegen seiner großen Wasserlöslichkeit leicht in den gewünschten Mengen zusetzen. Für die vollständige parenterale Ernährung sollte in der Infusionslösung vorzugsweise das Tripeptid entsprechend einer Menge von 0,5 bis 0,8 g Tyrosin je Liter ($2{,}762 \cdot 10^{-3}$ bis $4{,}420 \cdot 10^{-2}$ Mol) vorhanden sein. In einer 5%igen Aminosäurelösung sollte daher das $N^2$-Tyrosinyl-$N^6$-L-tyrosinyl-L-lysin in einer Menge von 0,6 bis 1,32 mMol 100 mL enthalten sein, um den biologisch und therapeutisch erforderlichen Mengen in der Infusionsmischung gerecht werden zu können. Wegen der guten Löslichkeit des Tripeptids kann erfindungsgemäß die erforderliche Menge an Tyrosin nun in solche Lösungen eingebracht werden.

Die nachfolgenden Beispiele 3—6 zeigen die Verwendung des erfindungsgemäßen $N^2$-Cystinyl-$N^6$-L-cystinyl-L-lysins (Cys-Lys(CyS)) und des $N^2$-Tyrosinyl-$N^6$-L-tyrosinyl-L-lysins (Tyr-Lys(Tyr)) in Kombination mit Aminosäuren und Peptiden bei Infusionslösungen. Das Tripeptid Glycyl-L-alanyl-L-glutamin (Gly-Ala-Gln) dient in der Lösung der Beispiele 5 und 6 als Glutaminquelle, wie dies in der gleichzeitig eingereichten EP-A2-0 087 750 näher beschrieben ist. Bei der Herstellung der Lösungen können außer den angegebenen Bestandteilen gegebenenfalls noch Mineralsalze, Vitamine oder dergl. in üblichen Mengen zugesetzt werden.

### Beispiel 3

Die erfindungsgemäßen beiden Tripeptide wurden zusammen mit den anderen Substanzen (essentielle Aminosäuren und Histidin) in Aqua dest. gelöst, die Lösung in Infusionsflaschen abgefüllt und sterilisiert (0,5 h bei 120° C).

| Peptide und Aminosäuren | g/1000 mL |
|---|---|
| Cys-Lys(CyS) | 3,1 |
| Tyr-Lys(Tyr) | 6,5 |
| L-Histidin | 5,5 |
| L-Isoleucin | 7,0 |
| L-Leucin | 11,0 |
| L-Lysin-Monoacetat | 7,7 |
| L-Methionin | 9,0 |
| L-Phenylalanin | 6,0 |
| L-Threonin | 5,0 |
| L-Tryptophan | 2,5 |
| L-Valin | 8,0 |

### Beispiel 4

| Peptide und Aminosäuren | g/1000 mL |
|---|---|
| Cys-Lys(CyS) | 2,8 |
| Tyr-Lys(Tyr) | 8,2 |
| L-Histidin | 4,9 |
| L-Isoleucin | 6,1 |
| L-Leucin | 9,6 |
| L-Lysin-Monoacetat | 6,63 |
| L-Methionin | 8,00 |
| L-Phenylalanin | 6,5 |
| L-Threonin | 5,7 |
| L-Tryptophan | 2,5 |
| L-Valin | 10,5 |

### Beispiel 5

| Peptide und Aminosäuren | g/1000 mL |
|---|---|
| Cys-Lys(CyS) | 3,5 |
| Tyr-Lys(Tyr) | 6,1 |
| L-Isoleucin | 6,0 |
| L-Leucin | 9,4 |
| L-Lysin-Monoacetat | 4,23 |
| L-Methionin | 6,5 |
| L-Phenylalanin | 6,0 |
| L-Threonin | 4,5 |

(Fortsetzung)

| Peptide und Aminosäuren | g/1000 mL |
|---|---|
| L-Tryptophan | 2,2 |
| L-Valin | 7,0 |
| L-Histidin | 4,5 |
| L-Alanin | 5,0 |
| L-Arginin | 5,0 |
| L-Glutaminsäure | 4,5 |
| Glycin | 1,0 |
| L-Serin | 2,5 |
| L-Prolin | 5,0 |
| Gly-Ala-Gln[1]) | 11,3 |

[1]) Tripeptid aus Glycin, L-Alanin und Glutamin.

Beispiel 6

| Peptide und Aminosäuren | g/1000 mL |
|---|---|
| CyS-Lys(CyS) | 0,9 |
| Tyr-Lys(Tyr) | 1,5 |
| L-Isoleucin | 2,51 |
| L-Leucin | 2,79 |
| L-Lysin | 1,45 |
| L-Methionin | 0,80 |
| L-Phenylalanin | 1,40 |
| L-Threonin | 1,74 |
| L-Tryptophan | 0,56 |
| L-Valin | 2,09 |
| L-Arginin | 3,49 |
| L-Histidin | 0,70 |
| L-Alanin | 5,50 |
| L-Asparagin | 4,0 |
| L-Glutaminsäure | 3,50 |
| Glycin | 0,50 |
| L-Prolin | 3,80 |
| Gly-Ala-Gln[1]) | 11,30 |

[1]) Tripeptid aus Glycin, L-Alanin und Glutamin.

**Patentansprüche**
**für die Vertragsstaaten CH, DE, GB, LI, SE**

1. Wasserlösliche Peptide sehr schwer löslicher Aminosäuren der allgemeinen Formel

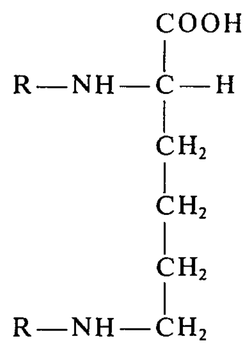

wobei R jeweils ein Tyrosinyl- oder Cystinylrest ist.

2. Verfahren zur Herstellung der Peptide nach Anspruch 1, dadurch gekennzeichnet, daß man Lysin und Tyrosin-N-Carboxy-Anhydrid oder Cystin-bis-N-Carboxy-Anhydrid im Mol-Verhältnis 1 : 1 bis 4 umsetzt, das Reaktionsprodukt durch Ansäuren decarboxyliert und $N^2$-Tyrosinyl-$N^6$-L-tyrosinyl-L-lysin oder das $N^2$-Cystinyl-$N^6$-L-cystinyl-L-lysin isoliert.

3. Verwendung der wasserlöslichen Peptide nach Anspruch 1 in oralen Aminosäure-Präparaten.

4. Verwendung der wasserlöslichen Peptide nach Anspruch 1 in Infusionslösungen für parenterale Ernährung.

5. Verwendung von $N^2$-Tyrosinyl-$N^6$-L-tyrosinyl-L-lysin in Mengen von 0,1—20 g, vorzugsweise 2—20 g, je Liter Infusionslösung.

6. Verwendung von $N^2$-Cystinyl-$N^6$-L-cystinyl-L-lysin in Mengen von 0,1—10 g, vorzugsweise 1—10 g, je Liter Infusionslösung.

**Patentansprüche für den Vertragsstaat AT**

1. Verfahren zur Herstellung wasserlöslicher Peptide sehr schwer löslicher Aminosäuren der allgemeinen Formel

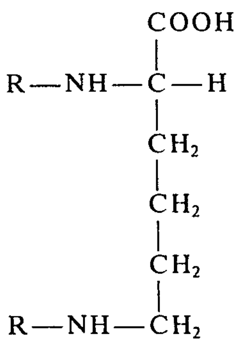

wobei R jeweils ein Tyrosinyl- oder Cystinylrest ist, dadurch gekennzeichnet, daß man Lysin und Tyrosin-N-Carboxy-Anhydrid oder Cystin-bis-N-Carboxy-Anhydrid im Mol-Verhältnis 1 : 1 bis 4 umsetzt, das Reaktionsprodukt durch Ansäuren decarboxyliert und $N^2$-Tyrosinyl-$N^6$-L-tyrosinyl-L-lysin oder das $N^2$-Cystinyl-$N^6$-L-cystinyl-L-lysin isoliert.

2. Verwendung der nach Anspruch 1 erhaltenen Peptide zur Herstellung oraler Aminosäurepräparate oder Infusionslösungen für parenterale Ernährung.

**Claims**
**for the Contracting States CH, DE, GB, LI, SE**

1. Water-soluble peptides of amino acids of very low solubility represented by the general formula

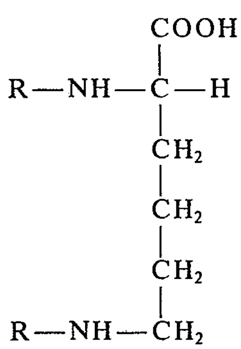

R being in each case a tyrosinyl or cystinyl group.

2. A process for producing the peptides as claimed in claim 1, characterized in that lysine and tyrosine-N-carboxy-anhydride or cystine-bis-N-carboxy-anhydride are reacted at a molar ratio of 1 : 1 to 4, the reaction product is decarboxylated by acidulation and $N^2$-tyrosinyl-$N^6$-L-tyrosinyl-L-lysine or $N^2$-cystinyl-$N^6$-L-cystinyl-L-lysine is isolated.

3. Use of the water-soluble peptides according to claim 1 in amino acid preparations to be adminstered orally.

4. The use of the water-soluble peptides according to claim 1 in infusion solutions for parenteral nutrition.

5. The use of $N^2$-tyrosinyl-$N^6$-L-tyrosinyl-L-lysine in quantities of 0.1 to 20 g, preferrably 2 to 20 g, per liter infusion solution.

6. The use of $N^2$-cystinyl-$N^6$-L-cystinyl-L-lysine in quantities of 0.1 to 10 g, preferrably 1 to 10 g, per liter infusion solution.

**Claims for the Contracting State AT**

1. A process for the production of water-soluble peptides of very low solubility represented by the general formula

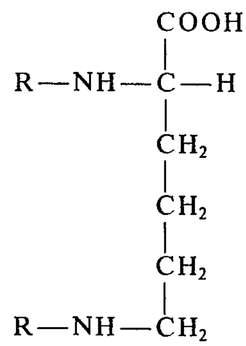

R being in each case a tyrosinyl or cystinyl group, characterized in that lysine and tyrosine-N-carboxy-anhydride or cystine-bis-N-carboxy-anhydride are reacted at a molar ratio of 1 : 1 to 4, the reaction product is decarboxylated by acidulation and $N^2$-tyrosinyl-$N^6$-L-tyrosinyl-L-lysine or $N^2$-cystinyl-$N^6$-L-cystinyl-L-lysine is isolated.

2. Use of the peptides obtained according to claim 1 for the production of amino acid preparation to be administered orally or infusion solutions for parenteral nutrition.

**Revendications**
**pour les Etats contractants CH, DE, GB, LI, SE**

1. Peptides solubles dans l'eau d'aminoacides très peu solubles répondant à la formule générale

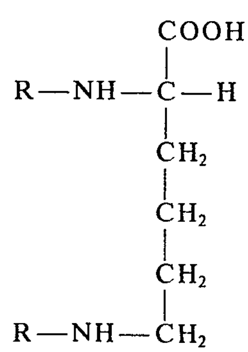

dans laquelle chaque R représente en un groupe tyrosinyle ou cystinyle.

2. Procédé de préparation des peptides selon la revendication 1, caractérisé en ce que l'on fait réagir de la lysine avec du N-carboxy-anhydride de tyrosine ou avec du bis(N-carboxy-anhydride) de cystine dans un rapport molaire compris entre 1 : 1 et 4, que l'on décarboxyle par acidulation le produit réactionnel et que l'on récupère la $N^2$-tyrosinyle-$N^6$-L-tyrosinyle-L-lysine ou la $N^2$-cystinyle-$N^6$-L-cystinyle-L-lysine.

3. Utilisation des peptides solubles dans l'eau selon la revendication 1 dans des préparations d'aminoacides pour application orale.

4. Utilisation des peptides solubles dans l'eau selon la revendication 1 dans des solutions pour infusion pour alimentation parenterale.

5. Utilisation de la $N^2$-tyrosinyle-$N^6$-L-tyrosinyle-L-lysine dans une quantité comprise entre 0,1 et 20 g, de préférence entre 2 et 20 g, par litre de solution pour infusion.

6. Utilisation de la $N^2$-cystinyle-$N^6$-L-cystinyle-L-lysine dans une quantité comprise entre 0,1 et 10 g, de préférence entre 1 et 10 g, par litre de solution pour infusion.

**Revendications pour l'Etat contractant AT**

1. Procédé de préparation de peptides solubles dans l'eau d'aminoacides très peu solubles, répondant à la formule générale

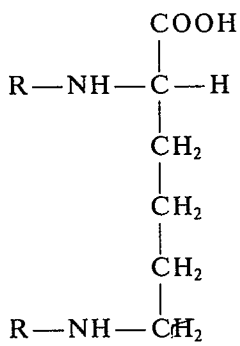

caractérisé en ce que l'on fait réagir de la lysine avec du N-carboxy-anhydride de tyrosine ou avec du bis(N-carboxy-anhydride) de cystine dans un rapport molaire compris entre 1 : 1 et 4, que l'on décarboxyle par acidulation le produit réactionnel et que l'on récupère la $N^2$-tyrosinyle-$N^6$-L-tyrosinyle-L-lysine ou la $N^2$-cystinyle-$N^6$-L-cystinyle-L-lysine.

2. Utilisation des peptides obtenus selon la revendication 1 pour la fabrication de préparations d'aminoacides pour application orale ou de solutions pour infusion pour alimentation parentérale.